# EUROPEAN PATENT APPLICATION

(11) **EP 4 205 631 A1**
(43) Date of publication of application: **05.07.2023**
(21) Application number: 20951211.0
(22) Date of filing: 02.12.2020
(51) Int. Cl.: A61B 1/313, A61B 1/06, A61B 1/04, A61B 34/30

(54) **ELECTRONIC ENDOSCOPE AND SURGICAL ROBOT**

(30) Priority: 31.08.2020 CN 202010899446
(71) Applicant: Shenzhen Jingfeng Medical Technology Co., Ltd., Shenzhen, Guangdong 518000 (CN)
(72) Inventor: WANG, Jianchen, Shenzhen, Guangdong 518000 (CN); CHEN, Zhengkun, Shenzhen, Guangdong 518000 (CN); HUANG, Jian, Shenzhen, Guangdong 518000 (CN)
(74) Representative: Zaboliene, Reda
(86) International application number: PCT/CN2020/133302
(87) International publication number: WO 2022/041543

(57) **Abstract**

An electronic endoscope (100) and a surgical robot. The electronic endoscope (100) includes an image capturing unit (12). The image capturing unit (12) includes: a housing (1); a lens base (2), mounted within the housing (1), the lens base (2) including a base body (21) and a thermally-conductive mesa structure (22) protrudingly provided on the base body (21); and a light source component (3), including a light source (32) provided on the thermally-conductive mesa structure (22), the thermally-conductive mesa structure (22) being used for conducting the heat generated by the light source (32). The design of the electronic endoscope (100) provides the light source (32) of the electronic endoscope (100) with excellent cooling, thus mitigating the problem of light attenuation.

## Description

The present disclosure is based on and claims priority to CN Patent Application No. 202010899446.8 filed on Aug. 31, 2020 in China National Intellectual Property Administration, entitled "ELECTRONIC ENDOSCOPE AND SURGICAL ROBOT", the contents of which are hereby incorporated by reference.

### FIELD

The present disclosure relates to medical devices and in particular to an electronic endoscope and a surgical robot.

### BACKGROUND

Minimally invasive surgery refers to a surgical method of performing a procedure in a human body cavity using modern medical instruments such as laparoscopes, thoracoscopes, and so on. Compared with traditional surgery modes, minimally invasive surgery has advantages of being small in trauma, light in pain, fast in recovery, and the like. At present, the endoscope used in minimally invasive surgery is mainly illuminated by light source(s) such as LED disposed at the front end of the endoscope and powered through a cable. However, due to the small size of the endoscope, it cannot provide good heat dissipation for the LED, so that the LED lighting cannot have higher luminance, which leads to light decay.

### SUMMARY

Aiming at the problems that the existing endoscope limited by size cannot provide good heat dissipation for the light source and thus leads to light decay, the main purpose of the present disclosure is to provide an electronic endoscope and a surgical robot.

To achieve the above purpose, the present disclosure provides an electronic endoscope. The electronic endoscope may include an image-capturing member, wherein the image-capturing member includes: a housing; a lens base, mounted in the housing, the lens base including a base body and at least one thermal-conductive mesa structure convexly provided on the base body; and a light source assembly, including at least one light source provided on the at least one thermal-conductive mesa structure, the at least one thermal-conductive mesa structure being configured to transfer heat generated by the at least one light source.

To achieve the above purpose, the present disclosure further provides a surgical robot. The surgical robot may include the above-mentioned electronic endoscope.

The electronic endoscope and surgical robot provided by some embodiments of the present disclosure can include an image-capturing member including: a housing; a lens base, mounted in the housing, the lens base including a base body and at least one thermal-conductive mesa structure convexly provided on the base body; and a light source assembly, including at least one light source provided on the at least one thermal-conductive mesa structure, the at least one thermal-conductive mesa structure being configured to transfer heat generated by the at least one light source. The heat generated by the light source(s) can be transferred to the end of the electronic endoscope which is further from the light source assembly via the thermal-conductive mesa structure(s) and the lens base. Thus, good heat dissipation can be provided for the light source(s) of the electronic endoscope, thereby reducing the light decay.

### BRIEF DESCRIPTION OF THE DRAWINGS

FIG. 1 is a partial exploded view of an electronic endoscope according to a first embodiment of the present disclosure;
FIG. 2 is an assembled view of the electronic endoscope in FIG. 1;
FIG. 3 is a partial assembled view of an electronic endoscope according to a second embodiment of the present disclosure;
FIG. 4 is a cross-sectional view of a complete structure of the electronic endoscope in FIG. 3;
FIG. 5 is an assembled view of an electronic endoscope according to a third embodiment of the present disclosure;
FIG. 6 is a top view of a lens base in FIG. 1;
FIG. 7 is a bottom view of the lens base in FIG. 1;
FIG. 8 is a partial schematic view of an embodiment of the electronic endoscope in any one of FIGS. 1-5;
FIG. 9 is a partial schematic view of another embodiment of the electronic endoscope in any one of FIGS. 1-5;
FIG. 10 is a schematic view of a first embodiment of the PCB of the electronic endoscope in any one of FIGS. 1-5;
FIG. 11 is a schematic view of a second embodiment of a PCB of the electronic endoscope in any one of FIGS. 1-5;
FIG. 12 is a schematic view of an embodiment of a shield of the electronic endoscope in any one of FIGS. 1-5;
FIG. 13 is a schematic view of an insulating cover of the electronic endoscope in any one of FIGS. 1-5;
FIG. 14 is a schematic view of a lens of the electronic endoscope in any one of FIGS. 1-5;
FIG. 15 is a schematic view of a lens cover and a lens of the electronic endoscope in any one of FIGS. 1-5;
FIG. 16 is a cross-sectional view of A-A portion of FIG. 2.
FIG. 17 is a partial assembled view of the electronic endoscope in FIG. 5;
FIG. 18 is a schematic view of the electronic endoscope in FIG. 17 from another viewing angel;
FIG. 19 is a partial exploded view of the electronic endoscope in FIG. 5;
FIG. 20 is an exploded view of a heat sink portion in FIG. 19.

### List of reference numbers:

| reference number | name | reference number | name | reference number | name |
|---|---|---|---|---|---|
| 100 | electronic endoscope | 1 | housing | 2 | lens base |
| 3 | light source assembly | 4 | lens cover | 5 | image sensor |
| 6 | PCB | 7 | shield | 8 | insulating cover |
| 11 | connecting portion | 21 | base body | 22 | thermal-conductive mesa structure |
| 23 | first mounting hole | 211 | first side | 212 | second side |
| 24 | mounting slot | 32 | light source | 33 | lens |
| 111 | wire passage | 25 | first extending portion | 26 | accommodating space |
| 60 | abutting surface | 221 | base plate | 222 | fixing slot |
| 223 | aluminum plate | 27 | central axis | 28 | wire hole |
| 61 | first board | 62 | second board | 71 | body |
| 72 | second extending portion | 73 | shielding plate | 74 | receiving space |
| 75 | through hole | 76 | thin wire hole | 81 | insulating cavity |
| 82 | insulating surface | 83 | hollow tube | 331 | lens body |
| 332 | stopping portion | 333 | first propping | 334 | second propping |
| | | | surface | | surface |
| 41 | cover body | 42 | receiving slot | 43 | second mounting hole |
| 411 | outer side surface | 412 | inner side surface | 413 | limiting part |
| 44 | fastening portion | 45 | containing space | 46 | optical filter |
| 421 | first slot | 422 | communicating slot | 423 | second slot |
| 9 | heat pipe portion | 10 | heat sink portion | 76 | outer wall |
| 77 | holding slot | 91 | first end | 200 | containing slot |
| 92 | second end | 93 | first heat pipe | 94 | second heat pipe |
| 781 | first outer wall | 782 | second outer wall | 771 | first holding slot |
| 101 | first heat sink | 102 | second heat sink | 103 | first containing slot |
| 104 | second containing slot | 105 | first sheet body | 107 | second sheet body |
| 112 | first extending sheet | 113 | second extending sheet | 109 | first wire groove |
| 110 | second wire groove | 12 | image-capturing member | 13 | joint assembly |
| 14 | driving member | 15 | tube body member | 70 | signal ground wire |
| 17 | connecting wire | 161 | internal thermal-conductive flexible tube | 162 | external thermal-conductive flexible tube |
| 18 | driving wire | 75' | through hole | 76' | thin wire hole |
| 115 | limiting portion | 116 | limiting groove | | |

The realization, functional features and advantages of the present application will be further described in conjunction with the embodiments and with reference to the accompanying drawings.

### DETAILED DESCRIPTION

The present disclosure will be described clearly and thoroughly herein by accompanying with appended figures of some embodiments. Apparently, the embodiments are only part of the present disclosure, and are not the whole disclosure. For a person skilled in the art, other embodiments may be obtained based on the provided embodiments without any creative work, and the other embodiments are also covered by the present disclosure.

The embodiments are described with reference to the accompanying drawings, in order to illustrate specific embodiments of the present disclosure that can be implemented. In the specification, it can be understood that, directional terms recited in the present disclosure, such as "top", "bottom", "upper", "lower", "front", "rear", "left", "right", "above", "under", and the like, only explain the relative positional relationship, motion, etc. between the various elements under a specific posture (as shown in the accompanying drawings). If the specific posture changes, the directional terms will change accordingly. Thus, the directional terms used here are only for better and more clearly describing and understanding the present disclosure, and are not intended to indicate or imply that the devices or the elements are disposed to locate at the specific directions or are structured and performed in the specific directions, which could not to be understood as limiting the present disclosure.

In the present application, unless otherwise specified and defined, the term " connected ", " fixed " and so on should be generalized understood. For example, unless there is a clear definition, " fixed " can be fixedly connected, detachably connected, or integrally connected; "connected" can be mechanically connected or electrically connected; "connected" can be directly connected, indirectly connected through the intermediate medium, inside of two elements communicates, or the interaction relation between two elements. For a person skilled in the art, it can be understood that the term in the present application of the specific meaning according to specific circumstances.

In addition, terms such as "first", "second", "third", and the like are used herein for purposes of description, and are not intended to indicate or imply relative importance or significance or to imply the number of indicated technical features. Thus, the feature defined with "first", "second", "third", and the like may include one or more of such a feature. Further, when "and/or" appears throughout the present disclosure, it means three concurrent solutions are included. For example, "A and/or B" includes solution A, or solution B, or a solution satisfying both A and B. Moreover, the embodiments and the features recited in the embodiments of the present disclosure may be combined with each other without confliction. However, the combination must base on the ability of realization by a person skilled in the art. When a combination is contradictory or cannot be realized, it should be considered that such combination does not exist and is not within the scope of protection claimed by the present disclosure.

The present application provides a surgical robot comprising a master operation console and a slave operation device. The master operation console may be configured to transmit control commands to the slave operation device according to the operation of the doctor to control the slave operation device. The slave operation device may be configured to perform corresponding surgical operations responding to the control commands from the master operation console. The slave operation device may comprise a mechanical arm, a power mechanism arranged on the mechanical arm, and an operating arm. The operating arm may be configured to enter the body driven by the power mechanism, perform surgical operations through end effector located at the distal end of the operating arm, and obtain intra-body images through electronic endoscope located at the distal end of the operating arm. The master operation console may be configured to display the images obtained by the electronic endoscope.

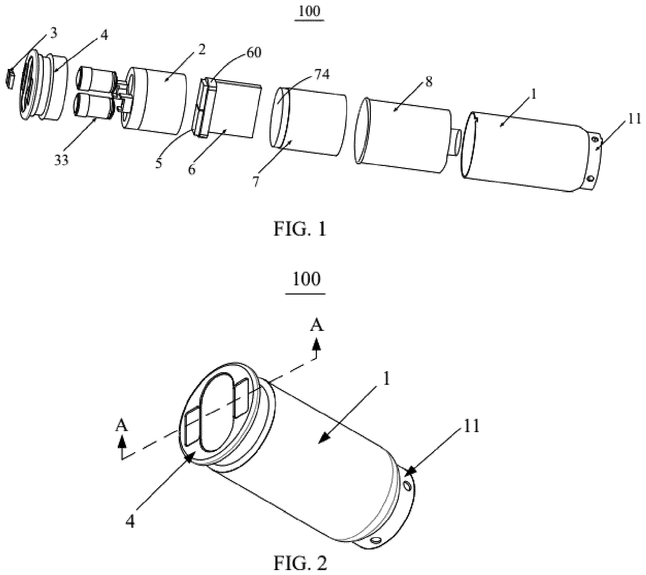

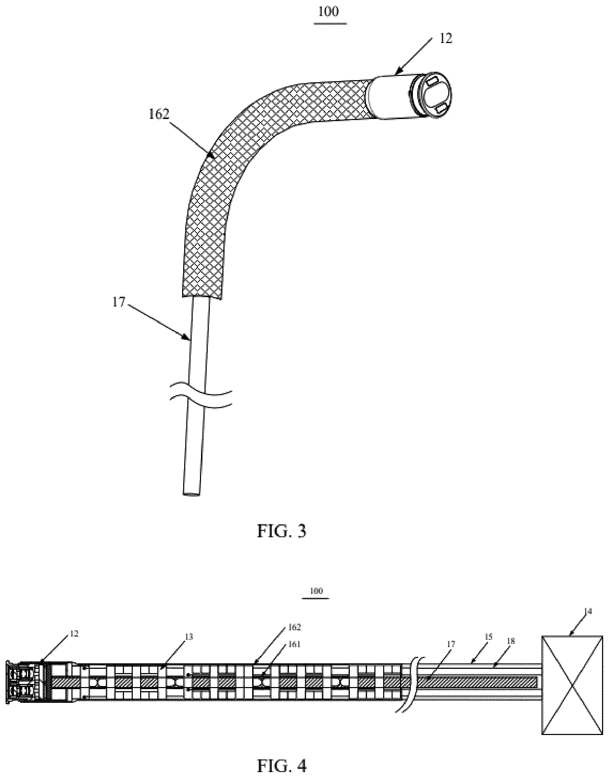

FIG. 1 and FIG. 2 illustrate an electronic endoscope 100 according to some embodiments of the present disclosure. The structure of the electronic endoscope 100 may be bendable or non-bendable. Referring to FIG. 3 and FIG. 4, when the structure of the electronic endoscope 100 is bendable, the electronic endoscope 100 may include an image-capturing member 12, a joint assembly 13 connected to the image-capturing member 12, an external thermal-conductive flexible tube 162 wrapping around the joint assembly 13 and connected to the image-capturing member 12, and a driving mechanism (not shown in figures) connected to the joint assembly 13.

Typically, referring to FIG. 4, the image-capturing member 12 may include a housing 1, a light source assembly 3 provided in the housing 1, a PCB 6, a connecting wire 17 connected to the PCB 6, and a connecting portion 11 connected to the joint assembly 13 and provided on the end of the housing 1 which is further from the light source assembly 3. The driving mechanism may comprise a driving member 14 and a plurality of driving wires 18 each having one end connected to the driving member 14 and the other end connected to the joint assembly 13. A first end of the connecting wire 17 may be connected to the PCB 6, a second end of the connecting wire 17 may be located in the driving member 14, and the part between the first end and the second end of the connecting wire 17 may be located in the joint assembly 13.

In some embodiments, the joint assembly 13 may be configured to be bent when driven by the driving member 14 and drive the image-capturing member 12 to move. In one embodiment, the external thermal-conductive flexible tube 162 may be connected to the housing 1. In another embodiment, the external thermal-conductive flexible tube 162 may be connected to the connecting portion 11. The heat generated by the light source assembly 3 can be transferred through the external thermal-conductive flexible tube 162 wrapping around the joint assembly 13.

Typically, the electronic endoscope 100 may further include an internal thermal-conductive flexible tube 161 wrapping around the connecting wire 17. The length of the internal thermal-conductive flexible tube 161 is not limited. The internal thermal-conductive flexible tube 161 may extend into the housing 1 to be connected to the PCB 6, or extend into the housing 1 without being connected to the PCB 6, or not extend into the housing 1. The specific configuration of the internal thermal-conductive flexible tube 161 can be reasonably selected according to practical requirements.

Typically, only the internal thermal-conductive flexible tube 161 may be provided, only the external thermal-conductive flexible tube 162 may be provided, or both the internal thermal-conductive flexible tube 161 and the external thermal-conductive flexible tube 162 may be provided. When both the internal thermal-conductive flexible tube 161 and the external thermal-conductive flexible tube 162 are provided, the heat dissipation of the electronic endoscope 100 can be significantly improved by the two-way thermal conductivity. Furthermore, the internal thermal-conductive flexible tube 161 and/or the external thermal-conductive flexible tube 162 may be mesh tube(s) made of woven metal wires. In other embodiments, the internal thermal-conductive flexible tube 161 and/or the external thermal-conductive flexible tube 162 may be made of other flexible materials with good thermal-conductivity.

Typically, a plurality of identical connecting units can be connected to form bendable joint assembly 13. The driving member 14 may be connected to the joint assembly 13 via the driving wires 18. The driving member 14 may drive the joint assembly 13 to bend by driving the elongation and contraction of the driving wires 18 connected to different connecting units in the joint assembly 13. The bending of the joint assembly 13 may drive the image-capturing member 12 to move.

In one embodiment, stretching springs (not shown in figures) each with a stretching direction set to be the same as the length direction of the driving wires 18 may be provided between the connecting units in the joint assembly 13 so as to improve the stiffness of the joint assembly 13.

In another embodiment, the electronic endoscope 100 may further include a tube body member15 with one end connected to the driving member 14 and the other end connected to the joint assembly 13. The driving wires 18 and the connecting wire 17 may pass through the tube body member 15 and be connected to the driving member 14. The stiffness of the joint assembly 13 can be improved by the support of the tube body member 15.

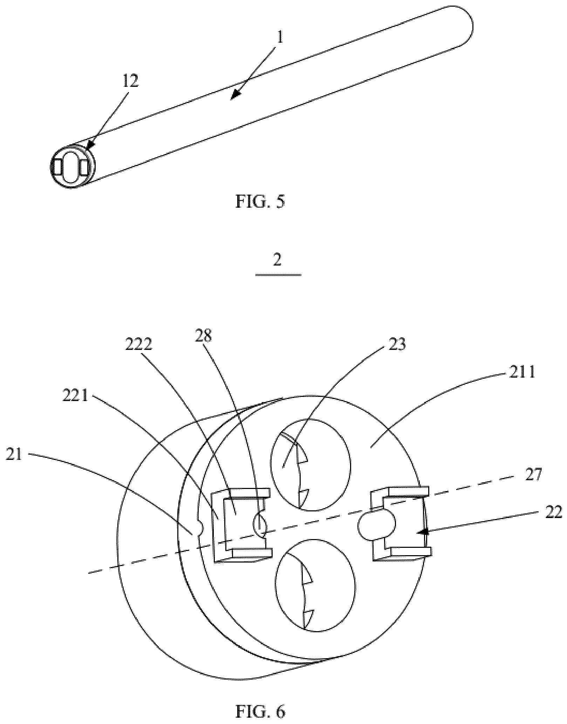

Referring to FIG. 5, when the structure of the electronic endoscope 100 is unbendable, the electronic endoscope 100 may include image-capturing member 12 without the joint assembly 13, the external thermal-conductive flexible tube 162 etc.. The electronic endoscope 100 may further include a rotation-driving member which drives the image-capturing member 12 to rotate about the central axis of the housing 1.

Typically, when the electronic endoscope 100 is bendable, it can be adapted to single-port surgery; when the electronic endoscope 100 is unbendable, it can be adapted to multiple-port surgery. However, the possible scenario that a bendable electronic endoscope is selected for multiple-port surgery cannot be excluded. The structure of the electronic endoscope 100 can be reasonably selected according to practical requirements.

Referring to FIG. 1, in one embodiment, the image-capturing member 12 may further include a lens base 2 mounted in the housing 1 and configured to mount the light source assembly 3, a lens cover 4 fastened to the lens base 2 and configured to accommodate the light source assembly 3, at least one image sensor 5 fixed in the lens base 2 and fixedly connected to the PCB 6 on the side closer to the light source assembly 3, a shield fastened to the lens base 2 on the side further from the lens cover 4, an insulating cover 8 sleeved on the lens base 2 and the shield 7. The housing 1 may be sleeved on the insulating cover 8. The above-described specific structure may form the front part of the image-capturing member 12. It can be understood that the holding space of the lens base 2 and the shield 7 can be filled with thermal-conductive adhesive so as to facilitate heat transfer.

In one embodiment, the internal thermal-conductive flexible tube161 may be connected to the shield 7 or extend into the thermal-conductive adhesive in the shield 7.

The structure of the housing 1 may be a hollow cylinder. In other embodiments, the structure of the housing 1 may be other reasonable structure. The housing 1 may be made of stainless steel etc.. It can be understood that when the structure of the housing 1 is the hollow cylinder, the structure of the lens cover 4, the lens base 2, the shield 7, the insulating cover 8 and etc. can be cylindrical structure adapted to the housing 1. In specific installation, the light source assembly 3, the image sensor(s) 5, and the PCB 6 can be fixed to the lens base 2 to form a first module (not shown in figures), then the lens cover 4 is fastened to the first module to form a second module (not shown in figures), then the shield 7 is fastened to the second module to form a third module (not shown in figures), and finally the housing 1 is fastened to the third module to form the electronic endoscope 100. Typically, the structure between neighboring modules can be tightly connected structure, e.g., the fixing in the above-mentioned installation can be interference fit. The adapted snap structure can be provided on the corresponding structure so as to further enhance the fixing strength between the modules. Thus, the tandem assembly of the modules can improve the assembling efficiency of the electronic endoscope 100. In other embodiments, part of the light source assembly 3 and the lens cover 4 can be assembled to form the first module (not shown in figures), other part of the light source assembly 3, the lens base 2, the image sensor(s) 5, the PCB 6 and the shield 7 can be assembled to form the second module (not shown in figures), the insulating cover 8 and the housing 1 can be assembled to form the third module (not shown in figures), and then the first module, the second module, and the third module can be assembled to form the electronic endoscope 100. Thus, the assembly efficiency of the electronic endoscope 100 can be improved by pre-assembling modules separately.

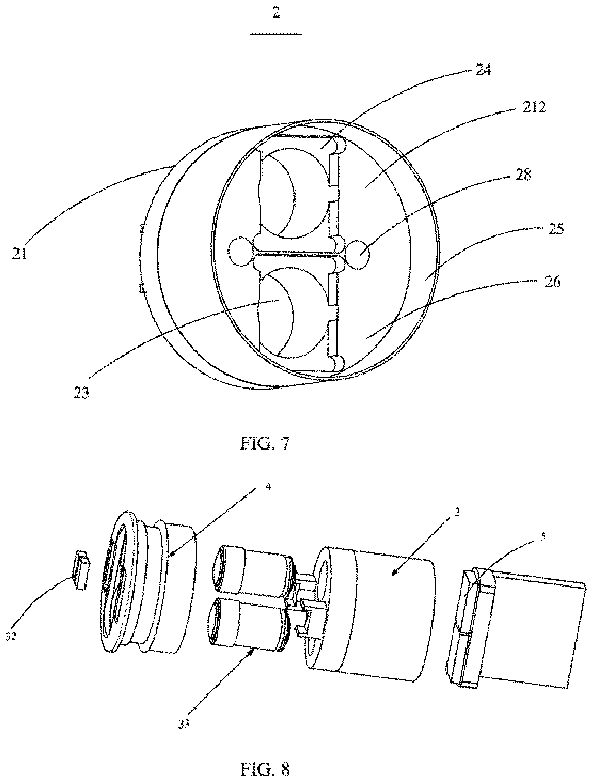

Referring to FIG. 6 and FIG. 7, the lens base 2 may include a base body 21, at least one thermal-conductive mesa structure(s) 22 convexly provided on the base body 21, at least one first mounting hole 23 provided on the base body 21, and at least one mounting slot 24 concavely provided on the base body 21. Typically, the base body 21 may be recessed from a side further from the thermal-conductive mesa structure(s) 22 toward the thermal-conductive mesa structure(s) 22 to define the mounting slot(s) 24. The position of the mounting slot(s) 24 may correspond to the position of the first mounting hole(s) 23. That is, the mounting slot 24 may communicate with the first mounting hole 23. Referring to FIG. 8, the light source assembly 3 may comprise at least one light source 32 provided on the base body 21 and at least one lens 33 configured to be mounted in the first mounting hole(s).

Furthermore, the image sensor(s) 5 may comprise a first image sensor (not shown in figures) and a second image sensor (not shown in figures). The mounting slot(s) 24 may comprise a first mounting slot (not shown in figures) configured to accommodate the first image sensor and a second mounting slot (not shown in figures) configured to accommodate the second image sensor. Correspondingly, the number of the first mounting hole(s) 23 may be two. The electronic endoscope 100 may further comprises a first lens corresponding to the first image sensor and a second lens corresponding to the second image sensor. The first lens and the second lens may be configured to be mounted in the two first mounting holes 23 respectively. When the first image sensor and the second image sensor are mounted in the first mounting slot and the second mounting slot respectively, a distance between the first lens and the first image sensor is equal to a distance between the second lens and the second image sensor.

In the installation of the electronic endoscope 100 according to the present embodiment, the two image sensor(s) 5 are first fixed to the PCB 6 and then mounted in the mounting slot(s) 24, which not only facilitates the installation of the two image sensor(s) 5, but also prevents inconsistencies of the installing positions between different image sensor(s) 5. Thus, inconsistencies of the images received by different image sensor(s) 5 can be avoided, thereby improving the safety of the doctor during the surgery using the electronic endoscope 100.

Referring to FIG. 6, the base body 21 may have a first side 211 and a second side 212 opposite to each other. Typically, when the light source(s) 32 is used as the reference, the first side 211 is the side closer to the light source(s) 32 and the second side 212 is the side further from the light source(s) 32; when the shield 7 is used as the reference, the first side 211 is the side further from the shield 7 and the second side 212 is the side closer to the shield 7. In one embodiment, the first side 211 can be connected to the thermal-conductive mesa structure(s) 22, the second side 212 can be the side further from the thermal-conductive mesa structure(s) 22 and opposite to the first side 211. The first mounting hole(s) 23 may each pass through the first side 211 and the second side 212. The base body 21 may be recessed from the second side 212 toward the first side 211 at the position corresponding to the first mounting hole(s) 23 to define the mounting slot(s) 24. The mounting slot(s) 24 may be configured to accommodate the image sensor(s) 5 respectively _{∘}

Typically, the light source(s) 32 can be provided on the thermal-conductive mesa structure(s) 22. The heat generated by the light source(s) 32 can be transferred through the base body 21 to the end of the electronic endoscope 100 which is further from the light source assembly 3. Thus, good heat dissipation can be provided for the light source(s) 32 of the electronic endoscope 100, thereby reducing the light decay.

It could be understood that the present disclosure does not limit the specific number of the lens(es) 33, which may be 2 or more. In other embodiments, there may be only one lens 33. Correspondingly, the number of the light source(s) 32, the number of the image sensor(s) 5, and the number of the lens(es) 33 are equal. In other embodiments, there may be only one image sensor(s) 5. However, compared with the scenario having only one image sensor 5, the present embodiment can avoid image interference and thus improve the quality of the image obtained by the electronic endoscope 100.

Typically, referring to FIG. 6, the base body 21 may extend from an edge of the second side 212 toward a direction away from the first side 211 to form a first extending portion 25. The first extending portion 25 may be enclosed with the second side 212 to define an accommodating space 26. Referring to FIGS. 1, 10 and 11, the PCB 6 may be provided with an abutting surface 60 adapted to the first extending portion 25. When the PCB 6 enters the accommodating space 26, the first extending portion 25 may contact the abutting surface 60. Thus, when the PCB 6 enters the accommodating space 26 through the guidance of the first extending portion 25, the PCB 6 and the image sensor(s) 5 can be accurately positioned and mounted in the mounting slot(s) 24, which can further improve the mounting accuracy of different image sensor(s) 5 as well as PCB 6 and the quality of images obtained by electronic endoscope 100.

Referring to FIG. 7, the diameter of the first mounting hole(s) 23 can be equal to the width of the mounting slot(s) 24. Alternatively, the width of the mounting slot(s) 24 may be greater than the diameter of the first mounting hole(s) 23, such that the fixing adhesive can be prevented from leaking to the lens(es) 33 during the module installation. When the image sensor(s)5 is fixed to mounting slot(s) 24 after being fixed to the PCB 6, the thermal-conductive adhesive can be filled in the accommodating space 26 and other holding spaces in a direction away from the lens cover 4, thus further increasing the thermal conductivity.

In one embodiment, referring to FIG. 6, the thermal-conductive mesa structure(s) 22 may each comprise a base plate 221 connected to the base body 21 and a fixing slot 222 concavely provided on the base plate 221. The base plate 221 may be recessed from a side further from the lens base 2 toward the lens base 2 to define the fixing slot 222. The fixing slot 222 may be configured to mount the light source(s) 32. Typically, the height of the light source(s) 32 may be equal to the height of the fixing slot 222, whereby the light source(s) 32 can be flush with the thermal-conductive mesa structure(s) 22.

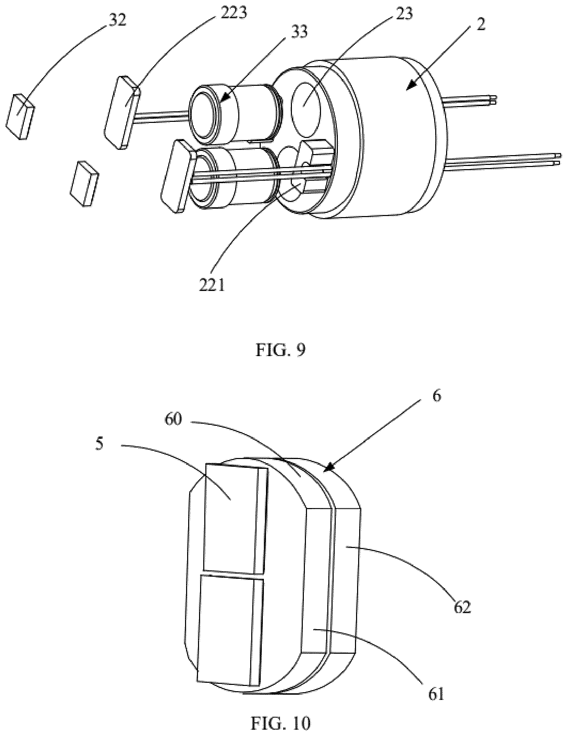

In another embodiment, referring to FIG. 9, the thermal-conductive mesa structure(s) 22 may include a base plate 221 connected to the base body 21 and an aluminum plate fixed to the base plate 221 and configured to mount and fix the light source(s) 32.

Typically, referring to FIG. 6, the lens base 2 has a central axis 27. At least one wire hole 28 may be provided on the lens base 2 for the wire of the light source(s) 32 to pass through. The wire hole(s) 28 may pass through the base plate 221 or be located between the thermal-conductive mesa structure(s) 22 and the central axis 27, so as to improve the overall strength of the lens base 2 compared with the scenario in which the wire hole(s) 28 is located on the outer side of the base body 21 and the edge of the base body 21 may be too thin.

The lens base 2 can be made of metal and non-metal material with good thermal conductivity. For instance, the lens base 2 can be made of copper or diamond, etc. Typically, the lens base 2 can be integrally formed.

Typically, the light source(s) 32 may be visible light, invisible light, or laser etc. light source. In the present embodiment, the light source(s) 32 may be LEDs emitting the visible light or optical fiber transmitting the visible light. When the LEDs are used for illumination, a matrix of LEDs may be mounted on the front end of the electronic endoscope 100, be supplied by a cable, and emit light to provide illumination for the endoscope. When the optical fiber is used for illumination, the optical fiber can be configured to transmit light from the remote end to the front end of the endoscope so that the endoscope is illuminated. The image sensor(s) 5 may be charge-coupled device (CCD) or complementary metal oxide semiconductor (CMOS) etc..

Referring to FIG. 10, the PCB 6 may be a stacked double-layer PCB. In another embodiment, the PCB 6 may have a T-shaped structure. Specifically, the PCB 6 may comprise a first board 61 fixed to the image sensor(s) and a second board 62 fixed to the first board 61. The plane in which the first board 61 is located is perpendicular to the plane in which the second board 62 is located.

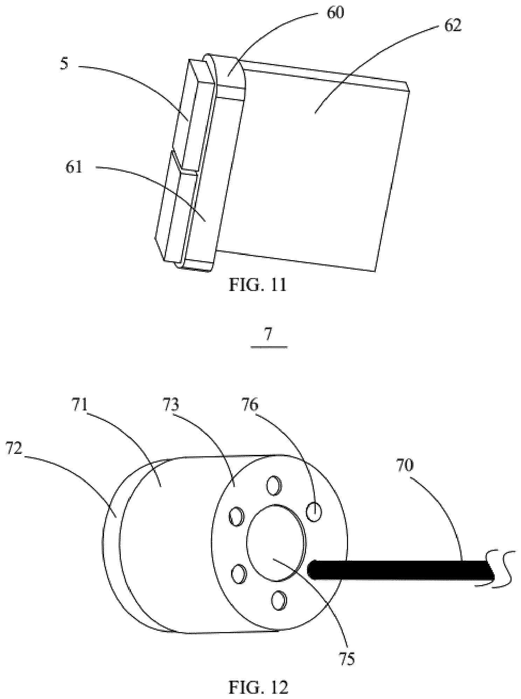

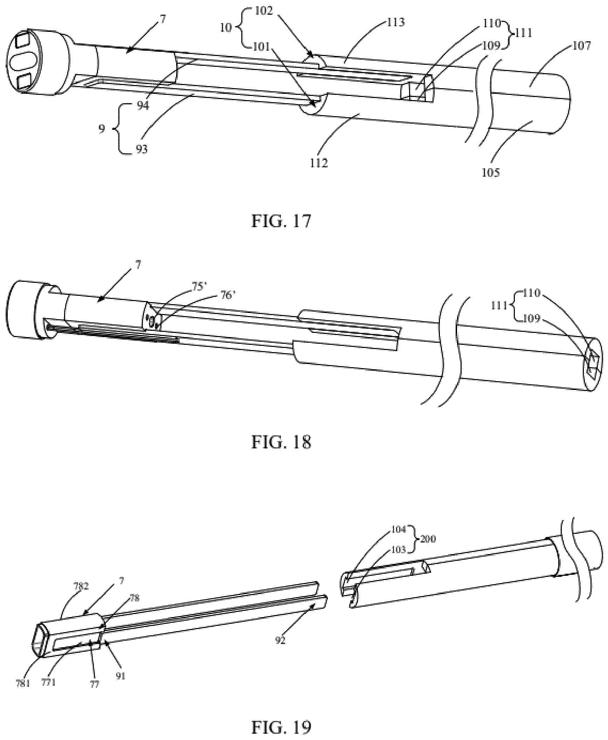

Referring to FIG.12, the shield 7 may comprise a body 71, a second extending portion 72 extending from the body 71 toward the lens base 2 and a shielding plate 73 located at an end of the body that is further from the lens base 2. The second extending portion 72 may be configured to be fastened to the first extending portion 25. Referring to FIG.1, the shield 7 can be enclosed with the lens base 2 to define a receiving space 74 configured to accommodate the PCB 6. Specifically, the second side 212, the first extending portion 25, and the shield 7 may be enclosed to define the receiving space 74 configured to accommodate the PCB 6. Referring to FIG.12, a through hole 75 for the connecting wire 17 and/or the connection wire of the light source(s) 32 to pass through may be provided on the shielding plate 73. The shield may be made of metal, which can produce a good electromagnetic shielding effect on the electronic components on the internal PCB 6, so as to avoid the electronic components being disturbed by the external complex electromagnetic environment, thereby ensuring stability and reliability of quality of images obtained by the electronic endoscope 100. Furthermore, at least one thin wire hole and signal ground wire(s) fixed in each thin wire hole may be provide on the shielding plate 73 near the through hole 75. The thin wire hole(s) 76 can be configured for the signal ground wire(s) 70 to pass through and be soldered in the thin wire hole(s) 76, which can further enhance the electromagnetic shielding effect of the shield 7. The number of the thin wire hole(s) 76 can be one, two, or more. In other embodiments, the signal ground wire(s) 70 can be soldered directly on the shielding plate 73 without the thin wire hole(s) 76. In another embodiments, referring to FIG.18, the shield 7 may be other shape and provided with the through hole 75' and thin wire hole(s) 76'.

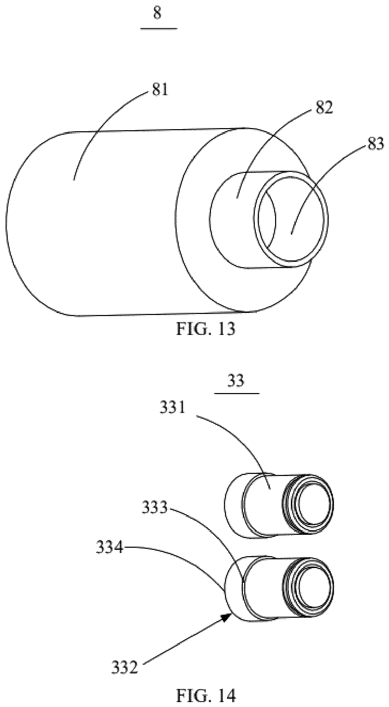

Referring to FIG.13, the insulating cover 8 may comprise an insulating cavity 81 connected to the shield 7 and the housing 1, an insulating surface 82 sealing the insulating cavity 81 and located in an end of the insulating cavity 81 which is further from the lens base 2, and a hollow tube 83 extending from the insulating surface 82 toward a direction away from the lens base 2. The hollow tube 83 may be configured to accommodate the connection wire of the light source(s) 32 and/or the connecting wire 17 connected to the PCB 6. Providing the hollow tube 83 can move the bending stress point of the connecting wire 17 toward a direction away from the lens base 2 and provide larger stress area and greater bending radius, so as to make the connection wire 17 more evenly stressed and thus less likely to break.

The insulating cover 8 may be located between the housing 1 and the lens base 2 and tightly connected to the housing 1 and the lens base 2. The heat generated by the light source(s) 32 can be transferred to the air via the lens base 2, the insulating cover 8, and the housing 1 sequentially. Since the shield 7 may be connected to the lens base 2, the insulating cover 8 may be located between the housing 1 and the shield 7 and tightly connected to the housing 1 and the shield 7, i.e., the insulating cover 8 may be sleeved on the lens base 2 and the shield 7, so as to avoid electric leakage of the electronic components of the electronic endoscope 100.

Referring to FIG.14, the lens(es) 33 each may comprise a lens body 331 with a central axis (not shown in figures) and a stopping portion 332. The lens body 331 may protrude from an end of the lens body 331 which is further from the lens base 2 toward a direction away from the central axis of the lens body 331 to form the stopping portion 332. The side of the stopping portion 332 which is closer to the lens base 2 may be provided with a first propping surface 333. The side of the stopping portion 332 which is further from the lens base 2 may be provided with a second propping surface 334. When the lens body 331 is mounted in the first mounting hole(s) 23 and contacts the lens base 2, the first propping surface 333 can be configured to limit the lens(es) 33 so as to prevent the lens(es) 33 from slipping out of the first mounting hole(s) 23.

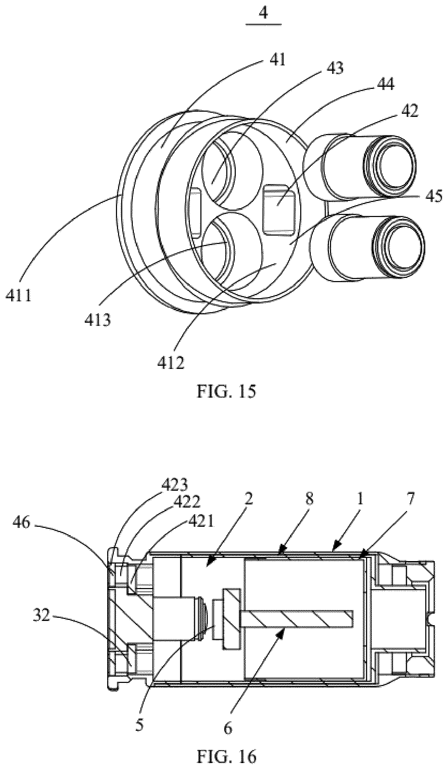

Referring to FIG.15, the lens cover 4 may comprise a cover body 41, at least one receiving slot 42 configured to accommodate the light source(s) 32 and the thermal-conductive mesa structure(s) 22, and at least one second mounting hole 43. The second mounting hole(s) 43 may be provided on the cover body 41 at the position corresponding to the first mounting hole(s) 23 respectively. The second mounting hole(s) 43 may be configured to accommodate the stopping portion 332. The cover body 41 may have an outer side surface 411 on the side further from the lens base 2 and an inner side surface 412 on the side closer to the lens base 2. The second mounting hole(s) 43 each may pass through the outer side surface 411 and the inner side surface 412. The receiving slot(s) 42 each may pass through the outer side surface 411 and the inner side surface 412.

The second mounting hole(s) 43 each may have a center (not shown in figures). The outer side surface 411 may extend at the position corresponding to each of the second mounting hole(s) 43 toward the center to form a limiting part 413. When the stopping portion 332 is mounted in the second mounting hole(s) 43, the limiting part 413 can contact the second propping surface 334 to limit the lens(es) 33 so as to prevent the lens(es) 33 from slipping out of the second mounting hole(s) 43.

The cover body 41 may extend from the edge of the inner side surface 412 toward the direction away from the outer side surface 411 to form a fastening portion 44. The fastening portion 44 can be enclosed with the inner side surface 412 to define a containing space 45. The fastening portion 44 may be fastened to the lens body 331 to accommodate the lens(es) 33, the light source(s) 32, and the thermal-conductive mesa structure(s) 22 in the containing space 45.

Referring to FIG.16, the lens cover 4 may further comprise at least one first cover glass (not shown in figures) capping the second mounting hole(s) 43 respectively and at least one second cover glass (not shown in figures) capping the receiving slot(s) 42 respectively. At least one optical filter 46 may be fixed above the light source(s) 32 and disposed between the second cover glass and the light source(s) 32. The first cover glass and the second cover glass can be transparent glass. Furthermore, sealing structures can be provided at the position of the gap between the first cover glass and the second mounting hole(s) 43 and the gap between the second cover glass and the first mounting hole(s) 23, so as to prevent the liquid from entering the electronic endoscope 100 through the gap and affecting stable operation of the electronic components. The sealing structure can be a sealing ring or a sealant.

Referring to FIG.16, the receiving slot(s) 42 each may further comprise a first slot 421 configured to accommodate the light source(s) 32, a communicating slot 422 communicating with the first slot 421 and disposed on the side further from the light source(s) 32, and a second slot 423 communicating with the communicating slot 422 and configured to accommodate the optical filter 46. The structural configuration of the receiving slot 42 can reduce the affect of the sinking design of the light source(s) 32 in the present application on the illuminance of the light source(s) 32.

Referring to FIGs.17-20, in one embodiment, the image-capturing member 12 may further comprise a heat pipe portion 9 connected to the shield 7 and a heat sink portion 10 connected to the heat pipe portion 9. The heat pipe portion 9 and the heat sink portion 10 may constitute the rear part of the image-capturing member 12. In other embodiments, the heat pipe portion 9 may extend to the lens base on one side of the shield 7. The lens base 2, the shield 7, the heat pipe portion 9, the heat sink portion 10, and the housing 1 can be made of materials with good thermal conductivity. The heat sink portion 10 may be tightly connected to the housing 1 so as to transfer the heat generated by the light source(s) 32 to the air via the lens base 2, the shield 7, the heat pipe portion 9, the heat sink portion 10, and the housing 1 sequentially. Thus, the temperature gradient between the light source(s) 32 and the housing 1 can be reduced by successively transferring the heat generated by the light source(s) 32 to the next structure, thereby reducing the temperature of the light source(s) 32.

In one embodiment, the image-capturing member 12 may comprise an outer wall (not shown in figures). A holding slot 77 can be concavely provided on the outer wall of the image-capturing member 12 and be configured to accommodate the first end of the heat pipe portion 9. A containing slot 200 may be concavely provided on the heat sink portion 10 and be configured to accommodate the second end of the heat pipe portion 9. It can be understood that the outer wall of the image-capturing member 12 may be the outer wall of the shield 7 or the outer wall of the lens base 2, i.e., the holding slot 77 can be provided on the shield 7 or the lens base 2. Correspondingly, the shield 7 may have a first outer wall and a second outer wall opposite to each other, or the lens base 2 may have a first outer wall and a second outer wall opposite to each other. The following embodiments may be described with the holding slot 77 provided on the shield 7 as an example.

The shield 7 may further comprise an outer wall 78 connected to the second extending portion 72 on one side and connected to the shielding plate 73 on the other side. The holding slot 77 can be concavely provided on the outer wall 78 and be configured to accommodate the first end 91 of the heat pipe portion 9. The containing slot 200 may be concavely provided on the heat sink portion 10 and be configured to accommodate the second end 92 of the heat pipe portion 9. The first end 91 is the end closer to the lens base 2 and the second end 92 is the end further from the lens base 2. The heat pipe portion 9 may contain a liquid refrigerant and the specific heat dissipation principle of the heat pipe portion 9 may be described as follows.

The liquid-gas-liquid conversion can be performed in the heat pipe portion 9 due to the liquid refrigerant. When the heat generated by the heat source is transferred to the first end 91 of the heat pipe through the lens holder 2 and the shield 7 sequentially, the liquid refrigerant at the first end 91 of the heat pipe may absorb the heat, convert into gas, and move along the length direction of the heat pipe toward the second end 92 of the heat pipe under the action of pressure. The heat can be continuously transferred from the heat source to the heat sink portion 10 during the movement of the gaseous refrigerant. When the gaseous refrigerant reaches the second end 92, the heat of the second end 92 may be reduced due to being taken away by the heat sink portion 10, such that the refrigerant can convert from the gaseous state to the liquid state at the second end 92 and then flow back to the first end 91. Thus, the heat pipe portion 9 can transfer heat in a circular manner.

In other embodiments, the heat pipe may be replaced by a copper pipe. The copper tube may only be used as a thermal-conductive material. The heat generated by the heat source may be transferred to the copper pipe through the lens holder 2 and the shield 7 sequentially, and then the copper pipe can transfer the heat to the heat sink portion 10, thereby reducing the heat of the heat source. The copper pipe may be a flexible copper pipe.

It can be understood that, when the holding slot 77 is provided on the shield 7, the first extending portion 25 of the lens base 2 can be recessed and snap-connected to the holding slot 77 adaptively to increase the fixing strength between the shield 7 and the lens base 2.

Furthermore, the heat pipe portion 9 may comprise two heat pipes opposite to each other: a first heat pipe 93 and a second heat pipe 94. The outer wall 78 may comprise a first outer wall 781 and a second outer wall 782 opposite to each other. The holding slot 77 may comprise a first holding slot 771 and a second holding slot 772. The first holding slot 771 may be concavely provided on the first outer wall 781. The second holding slot may be concavely provided on the second outer wall 782. The heat sink portion 10 comprise a first heat sink 101 and a second heat sink 102 opposite to each other. A first containing slot 103 may be concavely provided on the first heat sink 101. A second containing slot 104 may be concavely provided on the second heat sink 102. The first holding slot 771 may be configured to accommodate the first end 91 of the first heat pipe 93.The first containing slot 103 may be configured to accommodate the second end 92 of the first heat pipe 93. The second holding slot may be configured to accommodate the first end 91 of the second heat pipe 94.The second containing slot 104 may be configured to accommodate the second end 92 of the second heat pipe 94.

In other embodiments, the number of the heat pipes may be more than two. Correspondingly, the number of the heat sinks may be more than two. It can be understood that the lengths of the heat pipe portion 9 and the heat sink portion 10 can be can be reasonably set according to practical requirements. When the lengths are relatively long, the heat dissipation effect is relatively good.

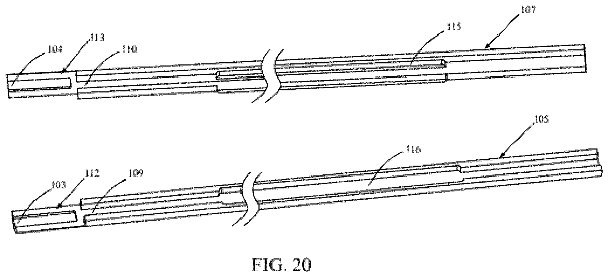

Furthermore, referring to FIG.20, the first heat sink 101 may comprise a first sheet body 105 and a first extending sheet 112 extending from the first sheet body 105 toward the lens base 2. The first containing slot 103 may be concavely provided on the first extending sheet 112. The second heat sink 102 may comprise a second sheet body 107 and a second extending sheet 113 extending from the second sheet body 107 toward the lens base 2. The second containing slot 104 may be concavely provided on the second extending sheet 113.

A first wire groove 109 may be concavely provided on an inner side of the first sheet body 105, and/or a second wire groove 110 may be concavely provided on an inner side of the second sheet body 107. The first wire groove 109 and/or the second wire groove 110 may be configured to accommodate the connecting wire 17 and/or the connection wire of the light source(s) 32. That is, only the first wire groove 109 or the second wire groove 110 may be provided, or the first wire groove 109 and the second wire groove 110 may be provided. Under the circumstance that the first wire groove 109 and the second wire groove 110 are provided, when the first sheet body 105 is mounted to the second sheet body 107, the first wire groove 109 and the second wire groove 110 may be combined to form a wire passage 111 for the connecting wire 17 and/or the connection wire of the light source(s) 32 to pass through.

Furthermore, referring to FIGS. 17-19, the shield 7 may be located between the first heat pipe 93 and the second heat pipe 94, the first heat pipe 93 and the second heat pipe 94 are located between the first heat sink 101 and the second heat sink 102. Thus, the fixed connection among the shield 7, the heat pipe portion 9 and the heat sink portion 10 can be realized without additional fixing structures.

Furthermore, referring to FIG. 20, a limiting portion 115 may be convexly provided on the second sheet body 107 toward the first sheet body 105. The first sheet body 105 may be provided with a limiting groove 116 corresponding to the limiting portion 115. The limiting portion 115 may be configured to limit movement of the second sheet body 107 in the direction toward or away from the lens base 2 when the limiting portion 115 is mounted in the limiting groove 116. It can be understood that the limiting groove 116 may be provided on the second sheet body 107 and the limiting portion 115 may be provided on the first sheet body 105. Furthermore, the limiting portion 115 is not limited to the manner of being convexly provided toward the first sheet body 105 in the present embodiment. The limiting portion 115 may be convexly provided toward the second wire groove 110 in other embodiments.

The heat generated by the light source(s) 32 can be transferred to the air via the heat pipe portion 9, the heat sink portion 10, and the housing 1 sequentially by providing the heat pipe portion 9 and the heat sink portion 10, thereby providing good heat dissipation for the electronic endoscope 100. When the heat generated by the light source(s) 32 is transferred to the end of the electronic endoscope 100 that is further from the light source assembly via the thermal-conductive mesa structure(s) 22 and the base body 21 of the image-capturing member 12, it can be selected that whether to provide the heat pipe portion 9 and the heat sink portion 10 according to practical requirements. For example, a flexibly bendable electronic endoscope 100 without the heat pipe portion 9 and the heat sink portion 10 can be selected for single-port surgery; an unbendable or bendable electronic endoscope 100 without the heat pipe portion 9 and the heat sink portion 10 can be selected for multiple-port surgery. Providing the heat pipe portion 9 and the heat sink portion 10 can further improve the heat dissipation of the electronic endoscope 100.

For a person skilled in the art, it is clear that the present disclosure is not limited to the details of the above exemplary embodiments, and that the present disclosure can be implemented in other specific forms without deviating from the spirit or basic characteristics of the application. Therefore, at any point, the embodiments should be regarded as exemplary and unrestrictive, and the scope of the present application is defined by the appended claims, rather than the above description. Therefore, all changes within the meaning and scope of the equivalent elements of the claim or directly/indirectly utilization in other related technical fields are intended to be included in protection scope of the present disclosure. Any reference number recited in the claims shall not be regarded as a limitation to the claims.

## Claims

1. An electronic endoscope comprising an image-capturing member, the image-capturing member comprising:
a housing;
a lens base mounted in the housing, the lens base comprising a base body and at least one thermal-conductive mesa structure convexly provided on the base body; and
a light source assembly comprising at least one light source provided on the at least one thermal-conductive mesa structure, the at least one thermal-conductive mesa structure being configured to transfer heat generated by the at least one light source.

2. The electronic endoscope of claim 1, **characterized in that**, the at least one thermal-conductive mesa structure each comprises a base plate connected to the base body and a fixing slot concavely provided on the base plate, the base plate is recessed from a side further from the lens base toward the lens base to define the fixing slot, and the fixing slot is configured to mount the at least one light source.

3. The electronic endoscope of claim 1, **characterized in that**, the image-capturing member further comprises at least one image sensor, the base body is recessed from a side further from the at least one thermal-conductive mesa structure toward the at least one thermal-conductive mesa structure to define at least one mounting slot, and the at least one mounting slot is configured to accommodate the at least one image sensor.

4. The electronic endoscope of claim 3, **characterized in that**, the light source assembly further comprises at least one lens mounted on the base body, the base body is provided with at least one first mounting hole configured to accommodate the at least one lens, the base body has a first side connected to the at least one thermal-conductive mesa structure and a second side opposite to the first side and further from the at least one thermal-conductive mesa structure, the at least one first mounting hole passes through the first side and the second side, and the at least one mounting slot communicates with the at least one first mounting hole.

5. The electronic endoscope of claim 4, **characterized in that**, the at least one image sensor comprises a first image sensor and a second image sensor, the at least one mounting slot comprises a first mounting slot configured to accommodate the first image sensor and a second mounting slot configured to accommodate the second image sensor; the at least one first mounting hole comprises two first mounting holes, the image-capturing member further comprises a first lens and a second lens, the first lens and the second lens are configured to be mounted in the two first mounting holes respectively; and the first image sensor and the second image sensor are configured to be mounted in the first mounting slot and the second mounting slot respectively, such that a distance between the first lens and the first image sensor is equal to a distance between the second lens and the second image sensor.

6. The electronic endoscope of claim 5, **characterized in that**, the image-capturing member further comprises a PCB provided in the housing, the base body extends from an edge of the second side toward a direction away from the first side to form a first extending portion, the PCB is provided with an abutting surface adapted to the first extending portion, and the first extending portion is configured to contact the abutting surface.

7. The electronic endoscope of claim 1, **characterized in that**, the image-capturing member further comprises a shield connected to the lens base, a first heat pipe connected to the shield, and a first heat sink connected to the first heat pipe; the shield has a first outer wall, a first holding slot is concavely provided on the first outer wall and configured to accommodate a first end of the first heat pipe, and a first containing slot is concavely provided on the first heat sink and configured to accommodate a second end of the first heat pipe.

8. The electronic endoscope of claim 7, **characterized in that**, the image-capturing member further comprises a second heat pipe connected to the shield and opposite to the first heat pipe and a second heat sink opposite to the first heat sink; the shield has a second outer wall opposite to the first outer wall, a second holding slot is concavely provided on the second outer wall and configured to accommodate a first end of the second heat pipe, and a second containing slot is concavely provided on the second heat sink and configured to accommodate a second end of the second heat pipe.

9. The electronic endoscope of claim 8, **characterized in that**, the first heat sink comprises a first sheet body and a first extending sheet extending from the first sheet body toward the lens base, the first containing slot is provided on the first extending sheet, the second heat sink comprises a second sheet body and a second extending sheet extending from the second sheet body toward the lens base, and the second containing slot is provided on the second extending sheet.

10. The electronic endoscope of claim 9, **characterized in that**, a first wire groove is concavely provided on an inner side of the first sheet body, and/or a second wire groove is being concavely provided on an inner side of the second sheet body, the electronic endoscope further comprises a PCB and a connecting wire connected to the PCB, and the first wire groove or the second wire groove is configured to allow the connecting wire to pass through.

11. The electronic endoscope of claim 10, **characterized in that**, a limiting portion is convexly provided on the second sheet body toward the first sheet body, the first sheet body is provided with a limiting groove corresponding to the limiting portion, and the limiting portion is configured to limit a movement of the second sheet body toward or away from the lens base when the limiting portion is mounted in the limiting groove.

12. The electronic endoscope of claim 8, **characterized in that**, the shield is located between the first heat pipe and the second heat pipe, and the first heat pipe and the second heat pipe are located between the first heat sink and the second heat sink.

13. The electronic endoscope of claim 1, **characterized in that**, the image-capturing member further comprises a shield fastened to the lens base and an insulating cover sleeved on the lens base and the shield, the housing is sleeved on the insulating cover, the insulating cover comprises an insulating cavity connected to the shield and the housing, an insulating surface sealing the insulating cavity and located in an end of the insulating cavity which is further from the lens base, and a hollow tube extending from the insulating surface toward a direction away from the lens base, and the hollow tube is configured to accommodate a connection wire of the at least one light source.

14. The electronic endoscope of claim 1, **characterized in that**, the light source assembly further comprises at least one lens mounted on the base body, the base body is provided with at least one first mounting hole configured to accommodate the at least one lens; the at least one lens each comprises a lens body with a central axis and a stopping portion, the lens body protrudes from an end of the lens body that is further from the lens base toward a direction away from the central axis to form the stopping portion, and a side of the stopping portion that is closer to the lens base is provided with a first propping surface, the first propping surface is configured to limit the lens when the lens body enters the first mounting hole and contacts the lens base.

15. The electronic endoscope of claim 14, **characterized in that**, the image-capturing member further comprises a lens cover fastened to the lens base, the lens cover comprises a cover body having an outer side surface on a side further from the lens base and an inner side surface on a side closer to the lens base, at least one second mounting hole is provided on the lens cover at a position corresponding to the at least one first mounting hole, the at least one second mounting hole is configured to accommodate the stopping portion and pass through the outer side surface and the inner side surface.

16. The electronic endoscope of claim 1, **characterized in that**, the image-capturing member further comprises a connecting portion provided on an end of the housing further from the lens base, the electronic endoscope further comprises a driving mechanism and a joint assembly connected to each of the connecting portion and the driving mechanism, the joint assembly is configured to be bent when driven by the driving mechanism and drive the image-capturing member to move, and the electronic endoscope further comprises an external thermal-conductive flexible tube connected to the housing, wrapped around the joint assembly and configured for heat transfer.

17. The electronic endoscope of claim 16, **characterized in that**, the image-capturing member further comprises a PCB provided in the housing and a connecting wire connected to the PCB, a first end of the connecting wire is located in the housing, a second end of the connecting wire is located in the driving mechanism, and the electronic endoscope further comprises an internal thermal-conductive flexible tube wrapped around the connecting wire.

18. The electronic endoscope of claim 17, **characterized in that**, the driving mechanism comprises a driving member and a driving wire having one end connected to the driving member and another end connected to the joint assembly; the second end of the connecting wire is located in the driving member, and a part of the connecting wire between the first end and the second end is located in the joint assembly.

19. The electronic endoscope of claim 18, **characterized in that**, the electronic endoscope further comprises a tube body member connected to the driving member at one end and connected to the joint assembly at another end, and the driving wire and the connecting wire both pass through the tube body member and are connected to the driving member.

20. A surgical robot, comprising an electronic endoscope, the electronic endoscope comprising an image-capturing member, the image-capturing member comprising:
a housing;
a lens base mounted in the housing, wherein the lens base comprises a base body and at least one thermal-conductive mesa structure convexly provided on the base body; and
a light source assembly comprising at least one light source provided on the at least one thermal-conductive mesa structure, wherein the at least one thermal-conductive mesa structure is configured to transfer heat generated by the at least one light source.
